# EUROPÄISCHE PATENTSCHRIFT

(11) **EP 0 994 675 B1**
(45) Veröffentlichungstag und Bekanntmachung des Hinweises auf die Patenterteilung: **05.11.2003**
(21) Anmeldenummer: 98932080.9
(22) Anmeldetag: 25.05.1998
(51) Int. Cl.: A61B 18/12

(54) **SCHUTZKAPPE FÜR MEDIZINISCHE HF-INSTRUMENTE**
PROTECTIVE CAP FOR MEDICAL HF INSTRUMENTS
CAPOT PROTECTEUR POUR INSTRUMENTS HF MEDICAUX

(30) Priorität: 27.05.1997 DE 19722063
(43) Veröffentlichungstag der Anmeldung: 26.04.2000
(73) Patentinhaber: Karl Storz GmbH & Co. KG, 78532 Tuttlingen (DE)
(72) Erfinder: DITTRICH, Horst, D-78194 Immendingen (DE); BACHER, Uwe, D-78532 Tuttlingen (DE)
(74) Vertreter: Weller, Wolfgang, Dr.
(86) Internationale Anmeldenummer: EP9803064
(87) Internationale Veröffentlichungsnummer: WO98053749

(56) Entgegenhaltungen:
- EP-A- 0 658 333
- DE-A- 4 236 394
- FR-A- 2 283 701
- US-A- 5 618 304
- US-A- 5 618 308

## Beschreibung

Die Erfindung betrifft eine Schutzkappe für medizinische HF-Instrumente, die das HF-Instrument zumindest in der Umgebung eines elektrischen Anschlusses für ein Hochfrequenzkabel isolierend abdeckt, wobei elektrisch beaufschlagte Bauelemente in der Umgebung des Anschlusses, die freiliegend sind, oder aufgrund der Betätigung des HF-Instruments freiliegend werden, mit abgedeckt sind.

Ein derartiges HF-Instrument ist aus der DE-A-4 236 394 bekannt. Darin ist ein Isolationssstück beschrieben, das das HF-Instrument in der Umgebung eines elektrischen Anschlusses isolierend abdeckt. Das Isolationsstück deckt elektrisch beaufschlagte, freiliegende Bauelemente in der Umgebung des HF-Anschlusses mit ab. Im Rahmen der Montage wird das Isolationsstück auf das HF-Instrument aufgesetzt und ist dann fester integraler Bestandteil des endfertigen Instrumentes.

HF-Instrumente dienen zur Hochfrequenzkoagulation oder zum Hochfrequenzschneiden.

Die Hochfrequenzkoagulation dient der operativen Zerstörung von Gewebezirken, zur Blutstillung, zur Abtragung von Gewebebereichen oder zur thermischen Koagulation von Tumoren.

Das Hochfrequenzschneiden kann mit den baugleichen HF-Instrumenten durchgeführt werden wie die Hochfrequenzkoagulation. Das Hochfrequenzschneiden wird bspw. zum Entfernen von Zysten, zum Durchtrennen von Gefäßen oder ähnlichen chirurgischen Eingriffen eingesetzt.

Zur Verwendung in der weit verbreiteten minimal-invasiven Chirurgie sind die HF-Instrumente als Rohrschaftinstrumente ausgebildet.

Rohrschaftinstrumente bestehen aus einem proximalen Handgriff und einem damit fest oder lösbar verbundenen Schaft, der als Rohrschaft ausgebildet ist. Durch diesen Rohrschaft wird ein Arbeitseinsatz geführt, der an seinem distalen Ende den Schaft überragt und das eigentliche Arbeitsgerät trägt, bspw. eine nadelförmige Elektrode oder Maulteile eines schneidenden oder fassenden Werkzeuges.

Der zu applizierende Strom wird von einem abseits des HF-Instrumentes angeordneten Hochfrequenzgenerator über ein Hochfrequenzkabel zugeführt. Für die elektrische Verbindung mit dem Instrument ist an diesem ein Anschluß vorgesehen. Dieser Anschluß besteht üblicherweise aus einem am Handgriff des Instrumentes angebrachten, von diesem abstehenden metallischen Stift.

Der Anschluß steht in elektrischem Kontakt mit dem eigentlichen Arbeitsgerät, so daß durch dieses der Strom bis an das distale Ende geführt wird.

Während der Anschluß für das Hochfrequenzkabel im allgemeinen fest mit dem HF-Instrument verbunden ist, wird der Generator über das Kabel nur bei Bedarf an das HF-Instrument angeschlossen.

Je nach Stromführung unterscheidet man zwischen unipolaren und bipolaren HF-Instrumenten.

Bei einem unipolaren, auch monopolaren genannten HF-Instrument ist an dem Instrument ein Anschluß nur für einen Pol vorgesehen. Der Patient liegt auf einer elektrisch leitenden Matte, die den zweiten Pol darstellt. Der vom distalen Ende des unipolaren HF-Instruments ausgehende Strom fließt breitflächig über den Körper des Patienten auf die leitende Matte ab.

Bei bipolaren HF-Instrumenten sind am Instrument zwei Anschlüsse vorhanden, d.h. der Strom wird am proximalen Ende eingespeist, einem Werkzeug, bspw. einer Koagulationsschlinge am distalen Ende zugeführt und dann wieder zum proximalen Ende zurückgeführt und dort wieder abgeführt.

Im folgenden umfaßt der Begriff "Anschluß" bei mono- oder unipolaren HF-Instrumenten einen Anschluß, bei bipolaren HF-Instrumenten zwei Anschlüsse bzw. Anschlußstecker.

Es wurde nun im praktischen Einsatz festgestellt, daß im Bereich des Anschlußsteckers elektrische Überschläge stattfinden können, die beim Operateur zu ruckartigen Reaktionen führen. Auch durch Kriechströme können Beeinträchtigungen auftreten.

Es wurden bisher verschiedene Lösungen vorgeschlagen, um den Operateur vor solchen Beeinträchtigungen zu schützen.

So ist in dem Katalog Endoskopische Chirurgie, Ausgabe 1/94 der Firma Karl Storz GmbH & Co. im Abschnitt 5 auf der Seite SCT 5/1 A ein Handgriff für ein Rohrschaftinstrument mit einem Anschluß für ein unipolares Hochfrequenzkabel dargestellt, bei dem ein Isolierung dadurch erreicht wird, daß der Handgriff im wesentlichen aus nicht leitendem Kunststoff besteht. Lediglich der Anschlußstift ist aus Metall und in den Kunststoff eingegossen. Der Handgriff weist ein festes und ein bewegliches Griffelement auf, die durch ein aus Kunststoff gefertigtes Scharniergelenk relativ zueinander bewegt werden können.

Diese Form der Isolierung hat den Nachteil, daß die Handgriffe keine ausreichende mechanische Stabilität aufweisen.

Die eingesetzten Scharnierbolzen aus Kunststoff waren den mechanischen Belastungen nicht gewachsen.

Daher wurden wieder metallische Gelenkbolzen eingesetzt.

Da die Gelenke jedoch in der näheren Umgebung des Anschlusses für das Hochfrequenzkabel liegen, besteht wieder die Gefahr von Spannungsüberschlägen oder Kriechströmen von dem Anschluß zu den metallischen Scharnierbolzen.

Wegen der geringen mechanischen Stabilität von Kunststoffgriffteilen für HF-Instrumente wurde alternativ vorgeschlagen, metallische Teile einzusetzen und diese mit einer elektrisch isolierenden Kunststoffschicht zu überziehen.

Diese Beschichtungen haben sich jedoch auf Dauer als nicht beständig gezeigt. Aufgrund der Handhabung, insbesondere beim Reinigen und Autoklavieren besteht die Gefahr, daß die Beschichtung beschädigt wird, sich ablöst und dadurch metallische Bereiche des Instrumentes freigelegt werden. Insbesondere kann eine Beschichtung dort, wo gegeneinander bewegliche Teile aneinanderstoßen, etwa an Gelenken, nicht vollständig deckend sein. Dadurch können dann beim HF-Einsatz Überschläge oder Kriechströme auftreten.

Der Handgriff am proximalen Ende des Instruments, an dem der Anschluß für das Hochfrequenzkabel vorhanden ist, dient dazu, das distale Ende des durch den Rohrschaft reichenden Arbeitsgerätes, z.B. spreizbare Maulteile, zu steuern.

Dazu steht ein Betätigungselement, meist in Form eines Stabes, proximal über den Rohrschaft hinaus und ist mit einem beweglichen Griffteil des Handgriffs verbunden. Bei den üblicherweise scherenartigen Handgriffen steht ein Ende des um die Scharnierachse beweglichen, also verschwenkbaren Griffteiles mit dem proximalen Ende des über den Rohrschaft überstehenden Betätigungselements in mechanischer Verbindung, um eine Schwenkbewegung des Griffteils in eine lineare Verschiebebewegung des stabförmigen Betätigungselementes umzusetzen.

Als mechanische Verbindung, insbesondere bei zerlegbaren Instrumenten, haben sich Kugelpfannengelenke durchgesetzt. Das üblicherweise blanke, proximal überstehende Ende des metallischen Betätigungselements liegt bauartbedingt in der Nähe des Anschlusses für das HF-Kabel und stellt im HF-Betrieb ebenfalls eine Ausgangsstelle für Überschläge dar. Selbst eine Isolierung dieses Abschnittes kann deswegen nicht abhelfen, da durch die reibende Kugelpfannenmechanik eine Isolierung alsbald abgerieben wäre.

Bei bipolaren HF-Instrumenten besteht außerdem die Gefahr von Überschlägen des Anschlußsteckers des einen Poles zum Anschlußstecker des anderen Poles.

Vor diesem Hintergrund ist es Aufgabe der vorliegenden Erfindung, eine sichere Isolierung für HF-Instrumente zu schaffen, die die Stabilität und Funktionsfähigkeit der übrigen Bauteile des HF-Instrumentes nicht beeinträchtigt und den Umgang mit dem HF-Instrument wesentlich erleichtert.

Erfindungsgemäß wird die Aufgabe durch eine Vorrichtung gemäß Anspruch 1 gelöst.

Durch das Vorsehen einer isolierenden Schutzkappe entfällt die unbedingte Notwendigkeit, das HF-Instrument oder zumindest diejenigen Bereiche des HF-Instrumentes, in denen der Anschluß für das Hochfrequenzkabel liegt, entweder vollständig aus isolierenden Materialien zu fertigen, oder bei metallischen Materialien diese durchgehend mit einer Isolierschicht zu versehen. So kann der Handgriff einschließlich seines Gelenkes und der Anschluß für das Hochfrequenzkabel aus Metall gefertigt sein und dennoch nach Aufbringen der Schutzkappe optimal elektrisch isoliert gegen Überschläge werden. Die Schutzkappe ermöglicht es somit, diejenigen Bauteile, die den elektrischen Anschluß enthalten, aus mechanisch stabilem und beständigem Material herzustellen. Da die Schutzkappe zumindest die Umgebung des elektrischen Anschlusses isolierend abdeckt, wird die Gefahr von Überschlägen oder Kriechströmen auf leitende Teile und damit auf den Operateur vermieden. Der elektrische Anschluß als solcher ist selbstverständlich gegenüber den Handgriffen elektrisch isoliert.

Je nach Ausgestaltung des Instrumentes als unipolares oder bipolares Instrument werden ein Anschlußstecker oder zwei Anschlußstecker bzw. deren Umgebung isolierend abgedeckt.

Die Schutzkappe kann als relativ preiswertes Bauteil aus einem beliebigen elektrisch gut isolierenden Material hergestellt werden. Bei der Fertigung als Spritzgußteil aus Kunststoff kann es z.B. zur Einmalverwendung ausgelegt sein.

Das HF-Instrument kann somit aus geeigneten, mechanisch stabilen Materialien hergestellt werden, ohne Rücksicht auf die Isolierung gegen Überschläge im Bereich des Anschlusses, da dies nunmehr durch die nachträglich oder im Bedarfsfall aufsetzbare Schutzkappe bewerkstelligt wird.

In einer weiteren Ausgestaltung der Erfindung werden elektrisch beaufschlagte Bauelemente in der Umgebung des Anschlusses, die freiliegend sind, oder aufgrund der Betätigung des HF-Instrumentes freiliegend werden, ebenfalls von der Schutzkappe mit abgedeckt.

Diese Maßnahme hat den erheblichen Vorteil, daß in allen möglichen Betriebs- und Handhabungsstellungen sichergestellt ist, daß nicht nur freiliegende metallische Teile sondern auch während der Handhabung freiwerdende metallische Teile abgedeckt sind. Bei der eingangs erwähnten Kugelpfannengelenkverbindung zwischen dem Betätigungselement im Rohrschaft und dem Griffteil wird, je nach Schwenkstellung des Griffteils, ein mehr oder wenig langer Abschnitt proximal aus dem Schaft herausgezogen. Da das Betätigungselement ja gerade zur elektrischen Leitung an das distale Ende herangezogen wird, ist dieses zwangsläufig aus metallisch leitendem Material hergestellt und stellt eine Ausgangsstelle für Überschläge oder Kriechströme dar. Es wird also sichergestellt, daß nicht nur Überschläge vom elektrischen Anschluß ausgeschlossen werden, sondern auch Beeinträchtigungen ausgehend vom proximal überstehenden metallisch leitenden Betätigungselement.

In einer weiteren Ausgestaltung ist die Schutzkappe auf das HF-Instrument aufsetzbar.

Diese Maßnahme hat den Vorteil, daß die Schutzkappe erst bei Bedarf, also beim HF-Betrieb aufgesetzt werden kann, ansonsten kann das Instrument ohne die Schutzkappe eingesetzt werden.

In einer weiteren Ausgestaltung der Erfindung ist die Schutzkappe auf einen Handgriff aufsetzbar, der über ein Scharniergelenk verbundene Griffteile aufweist, wobei sich die Schutzkappe über das Scharniergelenk hinweg erstreckt.

Diese Maßnahme hat nun den Vorteil, daß das Scharniergelenk unabhängig davon, ob die verbleibenden Griffelemente aus Kunststoffmaterial und anderen Materialien hergestellt wird, aus einem metallischen Bolzen bestehen kann, was erheblich zur mechanischen Stabilität des Griffes und des Gelenkes beiträgt. Die Schutzkappe erstreckt sich, ausgehend vom Anschluß, über den Bereich des Gelenkes hinweg, so daß Überschläge zum metallischen Gelenkbolzen ausgeschlossen sind.

In einer weiteren Ausgestaltung der Erfindung ist die Form der Schutzkappe der Kontur desjenigen Teils des HF-Instrumentes, auf den sie aufgesetzt wird, angepaßt.

Diese Maßnahme hat den Vorteil, daß sich die aufgesetzte Schutzkappe eng an die Kontur des HF-Instrumentes anschmiegt und kein sperriges, den Operateur störendes Bauteil darstellt. Somit wird ausgeschlossen, daß der Operateur wegen der aufgesetzten Schutzkappe das HF-Instrument anders ergreifen muß als im Nicht-HF-Betrieb ohne die Schutzkappe, was die Handhabungsfreundlichkeit und auch die Operationssicherheit begünstigt.

In einer weiteren Ausgestaltung der Erfindung ist die Form der Schutzkappe so ausgestaltet, daß eine Bewegung der Griffelemente im Bereich der Schutzkappe durch diese uneingeschränkt möglich ist.

Diese Maßnahme hat nun den Vorteil, daß die Schutzkappe den gesamten Bewegungsbereich der Griffelemente überdeckt, also in jeglicher Griffelementstellung eine ausreichende Isolierung sichergestellt ist, gleichzeitig die Bewegungsfreiheit des Griffelementes trotz aufgesetzter Schutzkappe nicht eingeschränkt ist.

In einer weiteren Ausgestaltung der Erfindung ist die Schutzkappe auf einen fest mit dem HF-Instrument verbundenen und von diesem abstehenden Anschluß für ein Hochfrequenzkabel aufschiebbar.

Diese Maßnahme hat den Vorteil, daß die Kappe sicher geführt über den Anschluß auf das Instrument aufgeschoben werden kann. Dieser Vorgang ist einfach, rasch und ohne große Aufmerksamkeit durchzuführen. In der Kappe kann dazu eine entsprechende Öffnung vorgesehen sein, durch die beim Aufschieben der vom Instrument abstehende Anschluß durchtritt, so daß dann die aufgeschobene Kappe unmittelbar den Anschluß umrundend abdecken kann, somit der Hauptgefahrenherd von Überschlägen besonders sicher isoliert ist.

In einer weiteren Ausgestaltung der Erfindung weist die Schutzkappe einen rohrförmigen Fortsatz auf, die über einen stiftförmigen, vom HF-Instrument abstehenden Anschluß schiebbar ist.

Diese Maßnahme hat den Vorteil, daß die Kappe durch den rohrförmigen Ansatz geführt zielgerecht in eine bestimmte Stellung auf das HF-Instrument aufgeschoben werden kann.

In einer weiteren Ausgestaltung der Erfindung ist ein Befestigungselement vorgesehen, mit dem die Schutzkappe auf dem HF-Instrument fixierbar ist.

Diese Maßnahme hat den Vorteil, daß die Schutzkappe besonders sicher und unverlierbar fixiert ist, so daß diese auch bei Ablegen des HF-Instrumentes zwischen mehreren Behandlungsvorgängen nicht verrutscht oder sich löst.

In einer weiteren Ausgestaltung der Erfindung besteht die Schutzkappe aus einem elektrisch isolierenden Kunststoffmaterial.

Diese Maßnahme hat den Vorteil, daß die Schutzkappe aus einem kostengünstigen Material herstellbar ist, das bspw. nach dem Spritzgußverfahren hergestellt werden kann, somit als Wegwerfteil für einmalige Benutzung vorgesehen werden kann.

In einer weiteren Ausgestaltung der Erfindung besteht die Schutzkappe aus einem keramischen Material, z.B. aus einer Oxidkeramik oder aus einer Glaskeramik.

Diese Maßnahme hat den Vorteil, daß ein nicht nur isolierendes sondern auch mechanisch äußerst widerstandsfähiges Material vorhanden ist, so daß die Schutzkappe auch zahlreichen Reinigungs- und Autoklaviervorgängen widersteht.

In einer weiteren Ausgestaltung der Erfindung ist die Schutzkappe lösbar auf das HF-Instrument aufsetzbar.

Diese Maßnahme hat den Vorteil, daß die Kappe entweder zum Reinigen oder, wenn das Instrument nicht mehr im HF-Betrieb eingesetzt wird, abgenommen werden kann.

In einer besonderen Ausgestaltung der Erfindung ist die Schutzkappe auf ein HF-Instrument aufsetzbar, bei dem ein Handgriff über eine lösbare Rastverbindung mit einem Rohrschaftelement verbunden ist, wobei ein Betätigungsknopf zum Schließen und Lösen der Rastverbindung vorhanden ist, wobei dieser Knopf zum Fixieren der Schutzkappe herangezogen wird.

Diese Maßnahme hat nun in diesem speziellen Fall den erheblichen Vorteil, daß mit einem an sich schon vorhandenen Bauelement am HF-Instrument zugleich die Fixierung der Schutzkappe bewerkstelligt wird. Für den Reinigungsvorgang kann das Instrument so zerlegt werden, daß die Schutzkappe als Einzelteil vorliegt und entsprechend gereinigt werden kann.

In einer weiteren Ausgestaltung der Erfindung weist die Schutzkappe selbst einen Anschluß für ein Hochfrequenzkabel auf, und der Anschluß steht nach dem Aufsetzen auf ein HF-Instrument mit einem leitenden Teil des HF-Instrumentes in Verbindung.

Diese Maßnahme hat nun den Vorteil, daß der Anschluß, der meist als metallischer Anschlußstift ausgebildet, in die Schutzkappe integriert ist, so daß bereits herstellerseits für eine entsprechend isolierende Verbindung zwischen diesen beiden Bauteilen gesorgt ist. Nach Aufsetzen der Schutzkappe wird durch den in dieser integrierten Anschluß die elektrische Verbindung zwischen dem HF-Anschlußkabel und dem leitenden Bauelement des HF-Instrumentes geschaffen. Bei einem bipolaren HF-Instrument sind dann zwei Anschlußstecker-Stifte vorhanden.

In einer weiteren Ausgestaltung der Erfindung ist vorgesehen, daß an der Schutzkappe Handhabungsmerkmale vorhanden sind, die ein Aufsetzen oder ein Abziehen der Schutzkappe erleichtern.

Diese Maßnahme hat den Vorteil, daß ein Auswechselvorgang einfach und sicher durchgeführt werden kann.

In einer weiteren Ausgestaltung der Erfindung umfassen die Handhabungsmerkmale Griffelemente, Griffrillen, einen angeformten Handgriff oder eine Aufrauhung.

Diese Maßnahmen sind zum einen fertigungstechnisch sehr einfach zu bewerkstelligen, insbesondere bei der Ausbildung als Spritzgußteil, andererseits bilden sie für die Finger einer menschlichen Hand eindeutige Handhabungsmerkmale, so daß ein sicheres Ergreifen und Abziehen bzw. Aufsetzen auch ohne hohe Aufmerksamkeit insbesondere ohne direkten Blickkontakt möglich ist.

In einer weiteren Ausgestaltung der Erfindung ist die Schutzkappe nur in einer bestimmten Stellung der Griffelemente auf das HF-Instrument aufbringbar bzw. von diesem abziehbar.

Diese Maßnahme hat den Vorteil, daß die Griffteile eine bestimmte Fixierung der Schutzkappe darstellen, ohne daß dafür ein zusätzliches Bedienelement notwendig wäre.

In einer weiteren Ausgestaltung der Erfindung ist die Schutzkappe mit dem Hochfrequenzkabel verbunden.

Diese Maßnahme hat den Vorteil, daß die Schutzkappe Teil des Steckers wird.

Die Erfindung wird nachfolgend anhand einiger ausgewählter Ausführungsbeispiele in Zusammenhang mit der beiliegenden Zeichnungen näher beschrieben und erläutert. Es zeigen:
- Fig. 1: eine perspektivische Explosionsdarstellung eines HF-Instrumentes und einer Schutzkappe in noch nicht aufgesetztem Zustand,
- Fig. 2: eine der Darstellung von Fig. 1 entsprechende Darstellung mit auf dem HF-Instrument aufgesetzter Schutzkappe, und
- Fig. 3: einen Längsschnitt eines weiteren Ausführungsbeispiels einer Schutzkappe mit integriertem Anschluß für ein HF-Kabel.

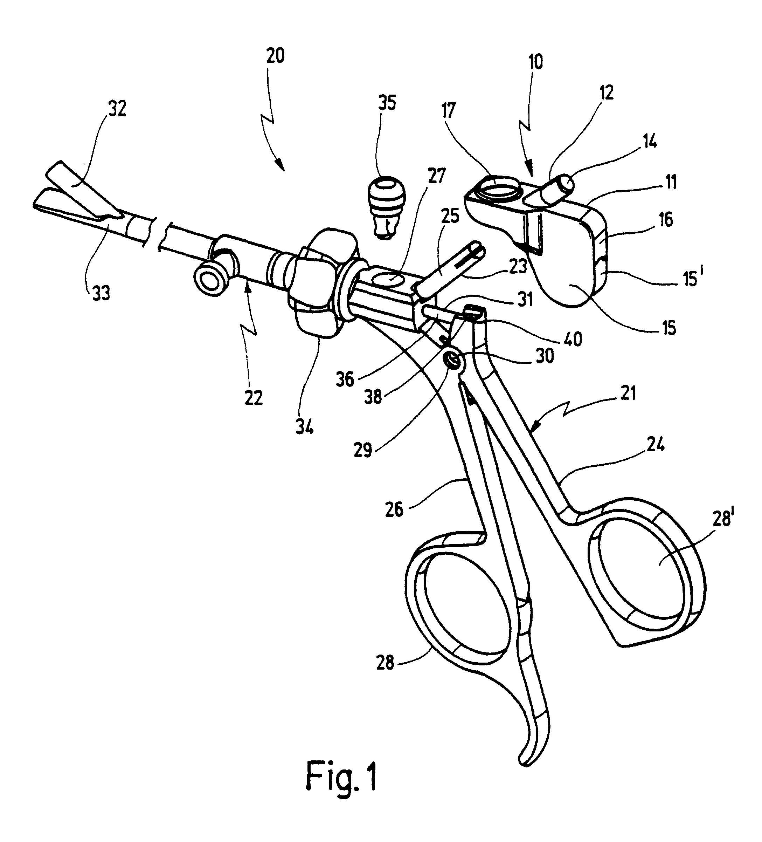

Eine in Fig. 1 gezeigte Schutzkappe 10 weist ein Gehäuse 11 auf, von dem ein rohrförmiger Ansatz 12 vorsteht.

Das Gehäuse 11 weist zwei Seitenwände 15, 15' auf, die über eine Querwand 16 miteinander verbunden sind.

Die Querwand 16 erstreckt sich über einen, in der Darstellung von Fig. 1 oberen Bereich und einen Teil des rückwärtigen proximalen Bereichs.

Vom oberen Bereich der Querwand 16 erstreckt sich der Ansatz 12 vor, der etwa um 45° zur Querwandfläche geneigt ist.

In der Querwand 16 ist an der Oberseite ferner eine Öffnung 17 vorgesehen. Die Öffnung 17 dient zur Aufnahme eines später beschriebenen Befestigungselementes, mittels dessen die Schutzkappe 10 an einem HF-Instrument 20 fixiert werden kann.

Die Schutzkappe 10 ist aus einer elektrisch isolierenden Oxidkeramik hergestellt.

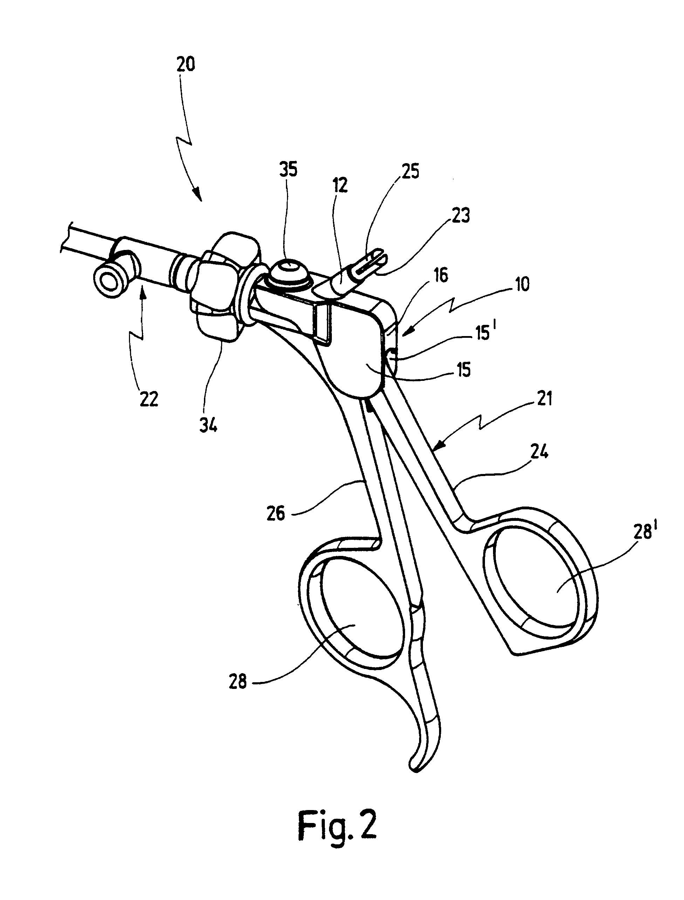

Das in Fig. 1 und 2 gezeigte unipolare HF-Instrument ist ein Rohrschaftinstrument mit einem Handgriff 21 und einem damit lösbar verbundenen Rohrschaft 22.

Der Handgriff 21 besteht aus einem beweglichen Griffelement 24 und einem festen Griffelement 26.

Das feste Griffelement 26 ist lösbar mit dem proximalen Ende des Rohrschaftes 22 verbunden.

Vom festen Griffelement 26 steht am proximalen Ende nach oben unter einem Winkel von 45° ein Anschluß 23 in Form eines metallischen Anschlußstiftes 25 vor.

Das bewegliche Griffelement 24 und das feste Griffelement 26 sind über ein Scharniergelenk 30 miteinander verbunden.

Ein Gelenkbolzen 29 des Scharniergelenks 30 stellt die Gelenkachse dar, um die das bewegliche Griffelement 24 relativ zum festen Griffelement 26 verschwenkt werden kann.

Beide Griffelemente 24 und 26 sind mit Fingerösen 28 bzw. 28' versehen.

Im Rohrschaft 22 ist ein stabförmiges Betätigungselement 31 aufgenommen, das den Rohrschaft 22 am distalen Ende überragt und zwei Maulteile 33 und 32 trägt.

Am proximalen Ende überragt ein Abschnitt 36 des Betätigungselementes 31 das proximale Ende des festen Griffelementes 26. Endseitig ist der Abschnitt 36 mit einer Kugel 38 versehen, die in einer entsprechenden Pfanne 40 am oberen Ende des beweglichen Griffelementes 24 aufgenommen ist.

Eine Verschwenkung des beweglichen Griffelementes 24 um das Scharniergelenk 30 bewerkstelligt eine lineare Verschiebung des Betätigungselementes 31 und damit ein Öffnen bzw. Schließen der Maulteile 32 bzw. 33.

Ein Stellrad 34 am festen Griffelement 26 dient dazu, den Rohrschaft 22 samt Betätigungselement 31 um die Rohrschaftachse zu drehen.

Ein Knopf 35 dient dazu, den Rohrschaft 22 axial unverschiebbar zu arretieren.

Der in etwa einem Winkel von 45° vom oberen Ende des festen Griffelementes 26 vorspringende Stift 25 kann durch den Innenraum 14 des rohrförmigen Ansatzes 12 der Schutzkappe 10 hindurchtreten.

Zum Aufsetzen der Schutzkappe 10 auf das unipolare HF-Instrument 20 wird diese, wie in Fig. 1 in der Explosionsdarstellung angesetzt, anschließend so aufgeschoben, daß der Stift 25 durch den rohrförmigen Ansatz 12 tritt.

Ist die Schutzkappe 10 vollständig aufgeschoben (siehe Fig. 2), kommt deren Öffnung 17 über derjenigen Öffnung 27 zum Liegen, in die der Knopf 35 mit Hilfe eines speziellen Werkzeuges (Spannzange) eingedreht werden soll.

Nun kann von der Außenseite her der Knopf 55 zunächst durch die Öffnung 17 in der Schutzkappe 10 durchgeschoben und anschließend in die Öffnung 27 eingesetzt und dort arretiert werden. Dadurch ist dann ein lösbarer, jedoch unverrückbarer Sitz der Schutzkappe 10 gewährleistet.

Wie aus Fig. 2 zu entnehmen, überragt der Stift 25 den rohrförmigen Ansatz 12, so daß auf diesen überragenden Abschnitt ein HF-Kabel aufgesetzt werden kann.

Aus der Darstellung von Fig. 2 ist auch zu entnehmen, daß der Abstand der Seitenwände 15, 15' so ist, daß gerade die Griffelemente 24 bzw. 26 dazwischen Platz haben.

Dadurch, daß die Querwand 16 sich auf der proximalen Seite nicht bis an das untere Ende der Schutzkappe 10 erstreckt, kann das bewegliche Griffelement 24 trotz aufgesetzter Schutzkappe 10 uneingeschränkt hin- und herbewegt werden.

Die Erstreckung der Seitenwände 15, 15', von dem Anschluß 23 in Richtung Fingerösen 28 gesehen, ist so, daß das Scharniergelenk 30 völlig abgedeckt ist. Dadurch ist es nicht möglich, daß Überschläge vom Anschluß 23 auf die Hand einer Person stattfinden können, die die Griffelemente ergriffen hat. Die Griffelemente 24 und 26 können entweder aus einem isolierenden Kunststoffmaterial oder aus einem mit einer isolierenden Kunststoffschicht überzogenen metallischen Material hergestellt sein.

Aus der Darstellung von Fig. 2 ist zu sehen, daß die Querwand 16 am oberen Ende den Bereich des Abschnittes 36 des Betätigungselementes 31 abdeckt, in dem dieses proximal das feste Griffelement 26 überragt.

Der Anschluß 23 steht elektrisch leitend mit dem Betätigungselement 31 in Verbindung, da über dieses der elektrische Strom an das distale Ende zu den Maulteilen 32 und 33 geleitet wird.

Durch diese Ausgestaltung und Geometrie der Schutzkappe 10 können im HF-Betrieb weder Überschläge von dem Anschluß 23 noch von dem überstehenden Abschnitt 36 auf den metallischen Bolzen 29 stattfinden, da dieser von der Schutzkappe 10 isolierend abgedeckt ist.

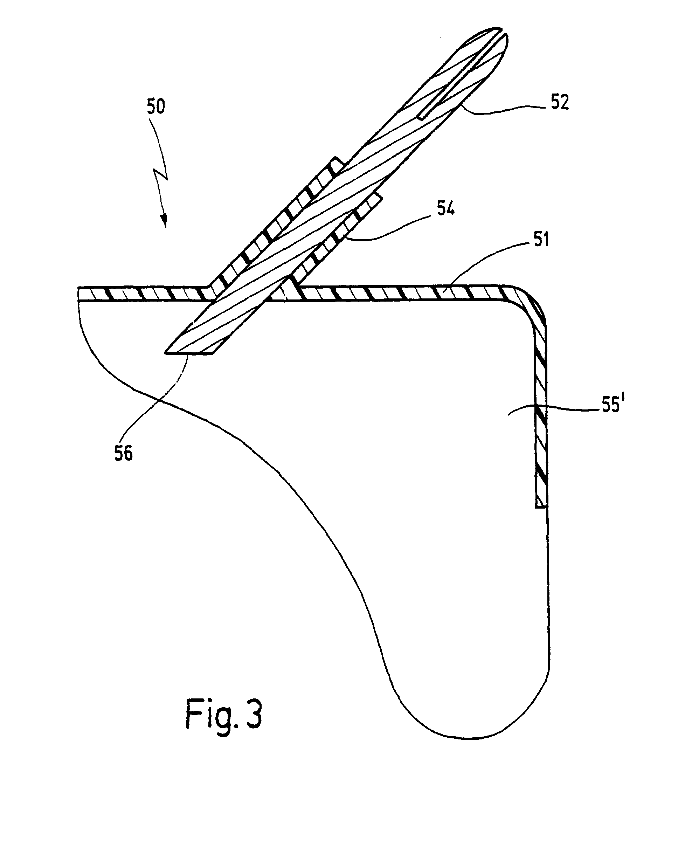

In Fig. 3 ist ein Längsschnitt eines weiteren Ausführungsbeispiels einer Schutzkappe 50 schematisch dargestellt.

Die Kontur der Schutzkappe 50 ist ähnlich der Kontur der zuvor beschriebenen Schutzkappe 10 ausgebildet, ein von der oberen Querwand 51 vorstehender rohrförmiger Ansatz 54 trägt einen metallischen Anschlußstift 52. Ein in das Innere der Schutzkappe 50 überstehendes Ende 56 des Anschlußstiftes dient dazu, in elektrischen Kontakt mit dem Betätigungselement zu treten, nachdem die Schutzkappe 50 auf ein HF-Instrument aufgesetzt wurde.

Im Gegensatz zu dem zuvor gezeigten Ausführungsbeispiel ist also der Anschlußstift 52 integraler Bestandteil der Schutzkappe 50. Bei einem bipolaren HF-Instrument sind dann zwei Anschlußstifte vorgesehen.

Im Gegensatz zu der zuvor dargestellten Ausführung der Schutzkappe 10 ist der Abstand der Querwände 51 so gewählt, daß diese unter Überwindung eines gewissen Reibwiderstandes gerade passend auf das feststehende Griffelement 26 aufgeschoben werden können. Es können dann auch noch zusätzliche Rast- oder Clipsvorrichtungen vorgesehen sein.

## Patentansprüche

1. Schutzkappe (10, 50) für medizinische HF-Instrumente (20), die das HF-Instrument (20) zumindest in der Umgebung eines elektrischen Anschlusses (23, 52) für ein Hochfrequenzkabel isolierend abdeckt, wobei elektrisch beaufschlagte Bauelemente in der Umgebung des Anschlusses (23, 52), die freiliegend sind oder aufgrund der Betätigung des HF-Instrumentes (20) freiliegend werden, mit abgedeckt sind, **dadurch gekennzeichnet, daß** die Schutzkappe (10, 50) bei Bedarf auf das HF-Instrument (20) aufsetzbar ist.

2. Schutzkappe nach Anspruch 1, **dadurch gekennzeichnet, daß** sie auf einen Handgriff (21) aufsetzbar ist, der über ein Scharniergelenk (30) verbundene Griffteile (24, 26) auf-weist, und wobei sich die Schutzkappe (10) über das Scharniergelenk (30) hinweg erstreckt.

3. Schutzkappe nach Anspruch 1 oder 2, **dadurch gekennzeichnet, daß** ihre Form der Kontur desjenigen Teils des HF-Instrumentes (20), auf den sie aufgesetzt ist, angepaßt ist.

4. Schutzkappe nach einem der Ansprüche 1 bis 3, **dadurch gekennzeichnet, daß** die Form der Schutzkappe (10, 50) so ausgestaltet ist, daß eine Bewegung von Griffteilen (24, 26) des HF-Instrumentes im Bereich der Schutzkappe durch diese uneingeschränkt möglich ist.

5. Schutzkappe nach einem der Ansprüche 1 bis 4, **dadurch gekennzeichnet, daß** sie auf einen fest mit dem HF-Instrument (20) verbundenen und von diesem abstehenden Anschluß (23) für ein Hochfrequenzkabel aufschiebbar ist.

6. Schutzkappe nach Anspruch 5, **dadurch gekennzeichnet, daß** sie einen rohrförmigen Fortsatz (12, 54) aufweist.

7. Schutzkappe nach einem der Ansprüche 1 bis 6, **dadurch gekennzeichnet, daß** ein Befestigungselement vorgesehen ist, mittels dessen die Schutzkappe auf dem HF-Instrument fixierbar ist.

8. Schutzkappe nach einem der Ansprüche 1 bis 7, **dadurch gekennzeichnet, daß** sie aus elektrisch isolierendem Kunststoff hergstellt ist.

9. Schutzkappe nach einem der Ansprüche 1 bis 7, **dadurch gekennzeichnet, daß** sie aus keramischem Material hergestellt ist.

10. Schutzkappe nach einem der Ansprüche 1 bis 9, **dadurch gekennzeichnet, daß** sie lösbar auf das HF-Instrument aufsetzbar ist.

11. Schutzkappe nach einem der Ansprüche 1 bis 10, **dadurch gekennzeichnet, daß** sie auf ein HF-Instrument (20) aufsetzbar ist, bei dem ein Handgriff (21) über eine Rastverbindung mit einem Rohrschaft (22) verbunden ist und die Schutzkappe (10) mit einem Knopf (35) zum Lösen der Rastverbindung fixierbar ist.

12. Schutzkappe nach einem der Ansprüche 1 bis 11, **dadurch gekennzeichnet, daß** die Schutzkappe (50) einen Anschluß (52) für ein Hochfrequenzkabel aufweist, und daß der Anschluß (52), nach dem Aufsetzen der Schutzkappe (50) auf ein HF-Instrument, mit einem leitenden Teil des HF-Instrumentes (20) leitend verbunden ist.

13. Schutzkappe nach einem der Ansprüche 1 bis 12, **dadurch gekennzeichnet, daß** Handhabungsmerkmale vorgesehen sind, die ein Ansetzen oder Abziehen der Schutzkappe erleichtern.

14. Schutzkappe nach Anspruch 13, **dadurch gekennzeichnet, daß** die Handhabungsmerkmale, Griffelemente, Griffrillen, einen angeformten Handgriff oder eine Aufrauhung umfassen.

15. Schutzkappe nach einem der Ansprüche 2 bis 14, **dadurch gekennzeichnet, daß** diese nur in einer bestimmten Stellung der Griffteile (24, 25) auf das HF-Instrument aufbringbar bzw. abziehbar ist.

16. Schutzkappe nach einem der Ansprüche 1 bis 15, **dadurch gekennzeichnet, daß** diese mit dem Hochfrequenzkabel verbunden ist.

17. Medizinisches HF-Instrument (20), **dadurch gekennzeichnet, daß** es eine Schutzkappe (10, 50) nach einem der Ansprüche 1 bis 16 aufgesetzt aufweist.

18. Medizinisches HF-Instrument nach Anspruch 17, **dadurch gekennzeichnet, daß** es zur Reinigung und Sterilisation zerlegbar ist.

## Claims

1. Protective cap (10, 50) for medical HF instruments (20), which covers the HF instrument (20) in insulating fashion at least in the vicinity of an electrical connector (23, 52) for a high-frequency cable, where current-carrying components in the vicinity of the connector (23, 52) that are exposed, or that become exposed due to actuation of the HF instrument (20), are also covered, **characterized in that** the protective cap (10, 50) can be placed on the HF instrument, if desired.

2. Protective cap of claim 1, **characterized in that** it can be placed onto a handle (21) that has handle elements (24, 26) joined via a hinge joint (30), the protective cap (10) extending over the hinge joint (30).

3. Protective cap of claims 1 or 2, **characterized in that** its shape is adapted to the contour of that part of the HF instrument (20) onto which it is placed.

4. Protective cap of anyone of claims 1 through 3, **characterized in that** the shape of the protective cap (10, 50) is configured such that movement of the handle elements (24, 25) of the HF instrument in the region of the protective cap is possible without being limited thereby.

5. Protective cap of anyone of claims 1 through 4, **characterized in that** it can be slid onto a connector (23) for a high-frequency cable, which connector (23) is joined immovably to the HF instrument (20) and protrudes therefrom.

6. Protective cap of claim 5, **characterized in that** it has a tubular extension (12, 54).

7. Protective cap of anyone of claims 1 through 6, **characterized in that** an attachment element is provided with which the protective cap can be secured on the HF instrument.

8. Protective cap of anyone of claims 1 through 7, **characterized in that** it is made of electrically insulating plastic.

9. Protective cap of anyone of claims 1 through 7, **characterized in that** it is made of ceramic material.

10. Protective cap of anyone of claims 1 through 9, **characterized in that** it can be placed onto the HF instrument in detachable fashion.

11. Protective cap of anyone of claims 1 through 10, **characterized in that** it can be placed onto an HF instrument (20) in which a handle (21) is joined to a tubular shaft element (22) via a snap-lock connection, and the protective cap (10) can be secured with a knob (35) for releasing the snap-lock connection.

12. Protective cap of anyone of claims 1 through 11, **characterized in that** the protective cap (50) has a connector (52) for a high-frequency cable; and **in that**, after the protective cap (50) has been placed onto an HF instrument, the connector (52) is conductively connected to a conductive part of the HF instrument (20).

13. Protective cap of anyone of claims 1 through 12, **characterized in that** handling features, which facilitate placement or removal of the protective cap, are provided.

14. Protective cap of claim 13, **characterized in that** the handling features comprise grip elements, grip ribs, a shaped-on handle, or a roughening.

15. Protective cap of anyone of claims 2 through 14, **characterized in that** it can be mounted onto the HF instrument and pulled off from it only when the grip elements (24, 25) are in a specific position.

16. Protective cap of anyone of claims 1 through 15, **characterized in that** it is joined to the high-frequency cable.

17. Medical HF instrument (20), **characterized in that** it has placed on it a protective cap (10, 50) as defined in anyone of claims 1 through 16.

18. Medical HF instrument of claim 17, **characterized in that** it can be disassembled for cleaning and sterilization.

## Revendications

1. Capuchon de protection (10, 50) pour instruments HF (20) médicaux, qui recouvre de manière isolante l'instrument HF (20) au moins dans la région d'une connexion électrique (23, 52) pour un câble à haute fréquence, des composants sollicités électriquement, dans la région de la connexion (23, 52), qui sont dégagés ou le deviennent par suite de l'actionnement de l'instrument HF (20), étant recouverts également, **caractérisé en ce que** le capuchon de protection (10, 50) peut être placé au besoin sur l'instrument HF (20).

2. Capuchon de protection selon la revendication 1, **caractérisé en ce qu'**il peut être placé sur une poignée (20) qui comporte des parties de poignée (24, 26) reliées par une articulation à charnière (30), et le capuchon de protection (10) s'étendant sur l'articulation à charnière (30).

3. Capuchon de protection selon la revendication 1 ou 2, **caractérisé en ce que** sa forme est adaptée au contour de la partie de l'instrument HF (20) sur laquelle il est placé.

4. Capuchon de protection selon l'une des revendications 1 à 3, **caractérisé en ce que** la forme du capuchon de protection (10, 50) est telle qu'un mouvement des parties de poignées (24, 26) de l'instrument HF est possible dans la zone du capuchon de protection, de manière non limitée par celui-ci.

5. Capuchon de protection selon l'une des revendications 1 à 4, **caractérisé en ce qu'**il peut être enfilé sur un raccordement (23), relié de manière fixe à l'instrument HF (20) et dépassant de celui-ci, destiné à un câble haute fréquence.

6. Capuchon de protection selon la revendication 5, **caractérisé en ce qu'**il présente un prolongement (12, 54) de forme tubulaire.

7. Capuchon de protection selon l'une des revendications 1 à 6, **caractérisé en ce qu'**il est prévu un élément de fixation au moyen duquel le capuchon de protection peut être fixé sur l'instrument HF.

8. Capuchon de protection selon l'une des revendications 1 à 7, **caractérisé en ce qu'**il est fabriqué dans une matière plastique isolante électriquement.

9. Capuchon de protection selon l'une des revendications 1 à 7, **caractérisé en ce qu'**il est fabriqué dans une matière céramique.

10. Capuchon de protection selon l'une des revendications 1 à 9, **caractérisé en ce qu'**il peut être placé de manière séparable sur l'instrument HF.

11. Capuchon de protection selon l'une des revendications 1 à 10, **caractérisé en ce qu'**il peut être placé sur un instrument HF (20) dans lequel une poignée (22) est reliée par une liaison à encliquetage, à une tige tubulaire (22), et le capuchon de protection (10) peut être fixé par un bouton (35) pour la suppression de la liaison à encliquetage.

12. Capuchon de protection selon l'une des revendications 1 à 11, **caractérisé en ce que** le capuchon de protection (50) comporte un raccordement (52) pour un câble à haute fréquence et **en ce que** le raccordement (52), après mise en place du capuchon de protection (50) sur un instrument HF, est relié de manière conductrice à une partie conductrice de l'instrument HF (20).

13. Capuchon de protection selon l'une des revendications 1 à 12, **caractérisé en ce que** sont prévues des caractéristiques de manipulation qui facilitent une mise en place ou un enlèvement du capuchon de protection.

14. Capuchon de protection selon la revendication 13, **caractérisé en ce que** les caractéristiques de manipulation comprennent des éléments de préhension, des gorges de préhension, une poignée venue de moulage ou un grattage.

15. Capuchon de protection selon l'une des revendications 2 à 14, **caractérisé en ce que** celui-ci ne peut être placé sur l'instrument HF ou retiré de celui-ci que dans une position déterminée des parties de poignée (24, 25).

16. Capuchon de protection selon l'une des revendications 1 à 15, **caractérisé en ce que** celui-ci est relié au câbie haute fréquence.

17. Instrument HF médical (20), **caractérisé en ce qu'**il comporte un capuchon de protection (10, 50) placé selon l'une des revendications 1 à 16.

18. Instrument HF médical selon la revendication 17, **caractérisé en ce qu'**il peut être démonté pour le nettoyage et la stérilisation.
